# EUROPEAN PATENT SPECIFICATION

(11) **EP 1 814 516 B1**
(45) Date of publication and mention of the grant of the patent: **26.12.2012**
(21) Application number: 06804325.6
(22) Date of filing: 03.11.2006
(51) Int. Cl.: A61K 9/00, A61K 47/02, A61K 47/10, A61K 39/35, A61K 39/36, A61P 37/08

(54) **USE OF A LIQUID ALLERGY VACCINE FORMULATION FOR OROMUCOSAL ADMINISTRATION**
VERWENDUNG EINER FORMULIERUNG EINES FLÜSSIGEN ALLERGIEIMPFSTOFFS ZUR VERABREICHUNG ÜBER DIE MUNDSCHLEIMHAUT
UTILISATION D UNE FORMULATION LIQUIDE DE VACCIN ANTIALLERGIQUE POUR ADMINISTRATION OROMUQUEUSE

(30) Priority: 04.11.2005 DK 200501526; 04.11.2005 US 733182 P
(43) Date of publication of application: 08.08.2007
(73) Proprietor: Alk-Abelló A/S, 2970 Hørsholm (DK)
(72) Inventor: HERNANDEZ, Domingo Barber, E-28027 Madrid (ES); MARTÍNEZ, Fernando de la Torre, E-28029 Madrid (ES); NIETO, Pilar Rico, Esc. Izda. 1· A (ES)
(74) Representative: Inspicos A/S
(86) International application number: PCT/DK2006/000609
(87) International publication number: WO 2007/051476

(56) References cited:
- WO-A-00/45847
- WO-A-02/40676
- WO-A-2005/077410
- US-A1- 2004 166 123
- FANTA C ET AL: "SYSTEMIC IMMUNOLOGICAL CHANGES INDUCED BY ADMINISTRATION OF GRASS POLLEN ALLERGENS VIA THE ORAL MUCOSA DURING SUBLINGUAL IMMUNOTHERAPY" INTERNATIONAL ARCHIVES OF ALLERGY AND IMMUNOLOGY, vol. 120, no. 3, November 1999 (1999-11), pages 218-224, XP008044918 ISSN: 1018-2438
- BUFE A ET AL: "Efficacy of sublingual swallow immunotherapy in children with severe grass pollen allergic symptoms: a double-blind placebo-controlled study" ALLERGY, MUNSKGAARD, COPENHAGEN, DK, vol. 59, no. 5, May 2004 (2004-05), pages 498-504, XP002334116 ISSN: 0105-4538

## Description

### TECHNICAL FIELD

The present invention relates to an allergy vaccine formulation for oromucosal administration.

### BACKGROUND OF THE INVENTION

Allergy is a major health problem in countries where Western lifestyle is adapted. Furthermore, the prevalence of allergic disease is increasing in these countries. Although allergy in general may not be considered a life-threatening disease, asthma annually causes a significant number of deaths. An exceptional prevalence of about 30% in teenagers conveys a substantial loss in quality of life, working days and money, and warrants a classification among major health problems in the Western world.

Allergy is a complex disease. Many factors contribute to the sensitisation event. Among these is the susceptibility of the individual defined by an as yet insufficiently understood interplay between several genes. Another important factor is allergen exposure above certain thresholds. Several environmental factors may be important in the sensitisation process including pollution, childhood infections, parasite infections, intestinal microorganisms, etc. Once an individual is sensitised and the allergic immune response established, the presence of only minute amounts of allergen is efficiently translated into symptoms.

The natural course of allergic disease is usually accompanied by aggravation at two levels. Firstly, a progression of symptoms and disease severity, as well as disease progression, for example from hay fever to asthma. Secondly, dissemination in offending allergens most often occurs resulting in allergic multi-reactivity. Chronic inflammation leads to a general weakening of the mucosal defense mechanisms resulting in unspecific irritation and eventually destruction of the mucosal tissue. Infants may become sensitised primarily to foods, i.e. milk, resulting in eczema or gastrointestinal disorders; however, most often they outgrow these symptoms spontaneously. These infants are at risk of developing inhalation allergy later in their lives.

The most important allergen sources are found among the most prevalent particles of a certain size in the air we breathe. These sources are remarkably universal and include grass pollens and house dust mite faecal particles, which together are responsible for approximately 50% of all allergies. Of global importance are also animal dander, i.e. cat and dog dander, other pollens, such as mugwort pollens, and micro-fungi, such as Alternaria. On a regional basis yet other pollens may dominate, such as birch pollen in Northern and Central Europe, ragweed in the Eastern and Central United States, and Japanese cedar pollen in Japan. Insects, i.e. bee and wasp venoms, and foods each account for approximately 2% of all allergies.

Allergy, i.e. type I hyper-sensitivity, is caused by an inappropriate immunological reaction to foreign non-pathogenic substances. Important clinical manifestations of allergy include asthma, hay fever, eczema, and gastro intestinal disorders. The allergic reaction is prompt and peaks within 20 minutes upon contact with the offending allergen. Furthermore, the allergic reaction is specific in the sense that a particular individual is sensitised to particular allergen(s), whereas the individual does not necessarily show an allergic reaction to other substances known to cause allergic disease. The allergic phenotype is characterized by a pronounced inflammation of the mucosa of the target organ and by the presence of allergen specific antibody of the IgE class in the circulation and on the surfaced of mast-cells and basophils.

An allergic attack is initiated by the reaction of the foreign allergen with allergen specific IgE antibodies, when the antibodies are bound to high affinity IgE specific receptors on the surface of mast-cells and basophils. The mast-cells and basophils contain preformed mediators, i.e. histamine, tryptase, and other substances, which are released upon cross-linking of two or more receptor-bound IgE antibodies. IgE antibodies are cross-linked by the simultaneous binding of one allergen molecule. It therefore follows that a foreign substance having only one antibody binding epitope does not initiate an allergic reaction. The cross-linking of receptor bound IgE on the surface of mast-cells also leads to release of signaling molecules responsible for the attraction of eosinophils, allergen specific T-cells, and other types of cells to the site of the allergic response. These cells in interplay with allergen, IgE and effector cells, lead to a renewed flash of symptoms occurring 12-24 hours after allergen encounter (late phase reaction).

Allergy disease management comprises diagnosis and treatment including prophylactic treatments. Diagnosis of allergy is concerned with by the demonstration of allergen specific IgE and identification of the allergen source. In many cases a careful anamnesis may be sufficient for the diagnosis of allergy and for the identification of the offending allergen source material. Most often, however, the diagnosis is supported by objective measures, such as skin prick test, blood test, or provocation test.

The therapeutic options fall in three major categories. The first opportunity is allergen avoidance or reduction of the exposure. Whereas allergen avoidance is obvious e.g. in the case of food allergens, it may be difficult or expensive, as for house dust mite allergens, or it may be impossible, as for pollen allergens. The second and most widely used therapeutic option is the prescription of classical symptomatic drugs like anti-histamines and steroids. Symptomatic drugs are safe and efficient; however, they do not alter the natural cause of the disease, neither do they control the disease dissemination. The third therapeutic alternative is specific allergy vaccination that in most cases reduces or alleviates the allergic symptoms caused by the allergen in question.

Conventional specific allergy vaccination is a causal treatment for allergic disease. It interferes with basic immunological mechanisms resulting in persistent improvement of the patients' immune status. Thus, the protective effect of specific allergy vaccination extends beyond the treatment period in contrast to symptomatic drug treatment. Some patients receiving the treatment are cured, and in addition, most patients experience a relief in disease severity and symptoms experienced, or at least an arrest in disease aggravation. Thus, specific allergy vaccination has preventive effects reducing the risk of hay fever developing into asthma, and reducing the risk of developing new sensitivities.

The immunological mechanism underlying successful allergy vaccination is not known in detail. A specific immune response, such as the production of antibodies against a particular pathogen, is known as an adaptive immune response. This response can be distinguished from the innate immune response, which is an unspecific reaction towards pathogens. An allergy vaccine is bound to address the adaptive immune response, which includes cells and molecules with antigen specificity, such as T-cells and the antibody producing B-cells. B-cells cannot mature into antibody producing cells without help from T-cells of the corresponding specificity. T-cells that participate in the stimulation of allergic immune responses are primarily of the Th2 type. Establishment of a new balance between Th1 and Th2 cells has been proposed to be beneficial and central to the immunological mechanism of specific allergy vaccination. Whether this is brought about by a reduction in Th2 cells, a shift from Th2 to Th1 cells, or an up-regulation of Th1 cells is controversial. Recently, regulatory T-cells have been proposed to be important for the mechanism of allergy vaccination. According to this model regulatory T-cells, i.e. Th3 or Tr1 cells, down-regulate both Th1 and Th2 cells of the corresponding antigen specificity. In spite of these ambiguities it is generally believed that an active vaccine must have the capacity to stimulate allergen specific T-cells, preferably TH1 cells.

Specific allergy vaccination is, in spite of its virtues, not in widespread use, primarily for two reasons. One reason is the inconveniences associated with the traditional vaccination programme that comprises repeated vaccinations i.a. injections over a several months. The other reason is, more importantly, the risk of allergic side reactions. Ordinary vaccinations against infectious agents are efficiently performed using a single or a few high dose immunizations. This strategy, however, cannot be used for allergy vaccination since a pathological immune response is already ongoing.

Conventional specific allergy vaccination is therefore carried out using multiple subcutaneous immunizations applied over an extended time period. The course is divided in two phases, the up dosing and the maintenance phase. In the up dosing phase increasing doses are applied, typically over a 16-week period, starting with minute doses. When the recommended maintenance dose is reached, this dose is applied for the maintenance phase, typically with injections every six weeks. Following each injection the patient must remain under medical attendance for 30 minutes due to the risk of anaphylactic side reactions, which in principle although extremely rare could be life-threatening. In addition, the clinic should be equipped to support emergency treatment. There is no doubt that a vaccine based on a different route of administration would eliminate or reduce the risk for allergic side reactions inherent in the current subcutaneous based vaccine as well as would facilitate a more widespread use, possibly even enabling self vaccination at home.

Attempts to improve vaccines for specific allergy vaccination have been performed for over 30 years and include multifarious approaches. Several approaches have addressed the allergen itself through modification of the IgE reactivity. Others have addressed the route of administration.

The immune system is accessible through the oral cavity and sublingual administration of allergens is a known route of administration. Administration may be carried out by placing the vaccine formulation under the tongue and allowing it to remain there for a short period of time, e.g. approx. 2 minutes.

Conventionally allergy vaccine using the oromucosal route consists of the up to daily dosing of a solution of the allergen. In comparison, the therapeutic (accumulated) maintenance doses given exceed the maintenance doses of a subcutaneous treatment by a high factor. Oromucosal administration is known to elicit no or only minor side effects, primarily in the form of itching in the mouth and sometimes itching in the eyes and ears.

WO 04/047794 discloses a solid fast-dispersing dosage form for sublingual administration of an allergy vaccine.

WO-A-00/ 45847 discloses an allergy vaccination for administration via a mucosal membrane of a subject; the oral route is clearly distinguished from the sublingual/ buccal route. The only explicit disclosure of a dosage regimen with no up-dosing is via peroral (direct delivery to the stomach) administration.

One commercial allergy vaccine for sublingual administration in the form of drops comprises an allergen dissolved in a liquid mixture of 50 % glycerol and 50 % water.

Commercial liquid allergy vaccines for sublingual administration are conventionally administered using a dosage regimen comprising a period of up-dosing, typically from 10 to 20 days of daily dosing, wherein the dose is gradually raised to the level of the maintenance dose, followed by a maintenance period, wherein the patient receives a maintenance dose. Recently, a number of studies have investigated the possibility of performing the up-dosing by way of a so-called rush up-dosing treatment effected over a few days, one day or even a few hours, wherein increasing doses are administered with only a short period of time, e.g. one hour or two hours, passing between each administration. However, such rush up-dosing treatment has the disadvantage that the occurrence of serious side effects cannot be ruled out, and hence like subcutaneous administration it requires the supervision of an allergy specialist.

The object of the present invention is to provide an improved use of preventing or treating allergy in a subject by oromucosal administration.

### SUMMARY OF THE INVENTION

This object is obtained by the present invention, which relates to the use of an allergen for the manufacture of a liquid vaccine formulation for preventing or treating allergy in a subject by oromucosal administration in a dosage regimen comprising no up-dosing, i.e. treatment may be initiated by the administration of a maintenance dose e.g. using the standard dosage regimen of the maintenance phase.

The present inventions is based on a clinical trial, which surprisingly has shown that allergy vaccination by oromucosal administration using a liquid formulation may be carried out with no up-dosing while maintaining an acceptable level of side effects. This finding is very surprising in the sense that it has hitherto been believed that an up-dosing phase of some kind is essential to avoid serious side effects.

The possibility of avoiding an up-dosing phase altogether provides oromucosal treatment with a liquid vaccine formulation with the advantage that it makes the dosage regimen much simpler. Furthermore, the simple dosage regimen increases the treatment compliance for the patient.

### DETAILED DESCRIPTION OF THE INVENTION

### Allergen

The allergen of the formulation according to the present invention may be any naturally occurring protein that has been reported to induce allergic, i.e. IgE mediated, reactions upon their repeated exposure to an individual. Examples of naturally occurring allergens include pollen allergens (tree-, herb, weed-, and grass pollen allergens), insect allergens (inhalant, saliva and venom allergens, e.g. mite allergens, cockroach and midges allergens, hymenopthera venom allergens), animal hair and dandruff allergens (from e.g. dog, cat, horse, rat, mouse etc.), and food allergens. Important pollen allergens from trees, grasses and herbs are such originating from the taxonomic orders of Fagales, Oleales, Pinales and platanaceae including i.a. birch (Betula), alder (Alnus), hazel (Corylus), hornbeam (Carpinus) and olive (Olea), cedar (Cryptomeria and Juniperus), Plane tree (Platanus), the order of Poales including i.a. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria . Other important inhalation allergens are those from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, and those from mammals such as cat, dog and horse, venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi and moulds are i.a. such originating from the genera Alternaria and Cladosporium.

In a particular embodiment of the invention the allergen is Bet v 1, Aln g 1, Cor a 1 and Car b 1, Que a 1, Cry j 1, Cry j 2, Cup a 1, Cup s 1, Jun a 1, Jun a 2, jun a 3, Ole e 1, Lig v 1, Pla l 1, Pla a 2, Amb a 1, Amb a 2, Amb t 5, Art v 1, Art v 2 Par j 1, Par j 2, Par j 3, Sal k 1, Ave e 1, Cyn d 1, Cyn d 7, Dac g 1, Fes p 1, Hol l 1, Lol p 1 and 5, Pha a 1, Pas n 1, Phl p 1, Phl p 5, Phl p 6, Poa p 1, Poa p 5, Sec c 1, Sec c 5, Sor h 1, Der f 1 , Der f 2, Der p 1, Der p 2, , Der p 7, Der m 1, Eur m 2, Gly d 1, Lep d 2, Blo t 1, Tyr p 2, Bla g 1, Bla g 2, Per a 1, Fel d 1, Can f 1, Can f 2, Bos d 2, Equ c 1, Equ c 2, Equ c 3 , Mus m 1, Rat n 1, Apis m 1, Api m 2 , Ves v 1, Ves v 2, Ves v 5, Dol m 1, Dil m 2, Dol m 5, Pol a 1, Pol a 2, Pol a 5, Sol i 1, Sol i 2, Sol i 3 and Sol i 4, Alt a 1, Cla h 1, Asp f 1, Bos d 4, Mal d 1, Gly m 1, Gly m 2, Gly m 3, Ara h 1, Ara h 2, Ara h 3, Ara h 4, Ara h 5 or shufflant hybrids from Molecular Breeding of any of these.

In a preferred embodiment of the invention the allergen is selected form the group consisting of a tree pollen allergen, a grass pollen allergen, a house dust mite allergen, a storage mite allergen, a weed allergen, a mould allergen, a cat allergen and a dog allergen.

In yet another embodiment of the invention the formulation comprises at least two different types of allergens either originating from the same allergic source or originating from different allergenic sources e.g. grass group 1 and grass group 5 allergens or mite group 1 and group 2 allergens from different mite and grass species respectively, weed antigens like short and giant ragweed allergens, different fungis allergens like alternaria and cladosporium, tree allergens like birch, hazel, hornbeam, oak and alder allergens, food allergens like peanut, soybean and milk allergens.

The allergen incorporated into the formulation may be in the form of an extract, a purified allergen, a modified allergen, a recombinant allergen or a mutant of a recombinant allergen. An allergenic extract may naturally contain one or more isoforms of the same allergen, whereas a recombinant allergen typically only represents one isoform of an allergen. In a preferred embodiment the allergen is in the form of an extract. In another preferred embodiment the allergen is a recombinant allergen. In a further preferred embodiment the allergen is a naturally occurring low IgE-binding mutant or a recombinant low IgE-binding mutant. Furthermore, the allergen may be a mixture of a number of allergens, e.g. from 2 to 10 allergens, in particular from 3 to 9 allergens, more in particular from 4 to 8, and most in particular from 5 to 7 allergens.

Allergens may be present in equi-molar amounts or the ratio of the allergens present may vary preferably up to 1:20.

In a further embodiment of the invention the low IgE binding allergen is an allergen according to WO 99/47680, WO 02/40676 or WO 03/096869 A2.

The preferred potency of a mono-dose formulation intended for daily administration is from 1 to 2000 STU, more preferable from 50 to 1000 STU, more preferably from 100 to 500 STU, more preferable from 130 to 350 STU and most preferable from 150 to 250 STU.

The preferred potency of a mono-dose formulation intended for daily administration is from 100 to 500000 SQ-u, more preferably from 200 to 300000 SQ-u, more preferable from 500 to 200000 SQ-u, more preferably from 1000 to 100000 SQ-u, more preferable from 1500 to 80000 SQ-u and most preferable from 2000 to 50000 SQ-u.

The amount of allergen, which corresponds to a given level of potency, varies strongly depending on the allergen specie. In a further embodiment of the invention the concentration of major allergen in a mono-dose intended for daily administration is from 0.05 to 50 µg, more preferably from 0.05 µg to 30 µg, more preferably from 0.06 µg to 25 µg, more preferably from 0.07 µg to 20 µg, more preferably from 0.08 µg to 15 µg, more preferably from 0.09 µg to 10 µg and most preferably from 0.1 µg to 7 µg.

### Oromucosal administration

The immune system is accessible through the oral cavity and sublingual administration of allergens is a known route of administration. Administration may be carried out by placing the vaccine formulation under the tongue and allowing it to remain there for a short period of time, e.g. from 30 to 300 seconds, preferably from 45 to 240 seconds, more preferably from 60 to 180 seconds, more preferably from 90 to 150 seconds, and most preferably from 90 to 120 seconds.

Oromucosal administration comprises any administration method, wherein the formulation in part or in full comes into contact with the mucosa of the oral cavity and/or the pharynx of the patient. Oromucosal administration methods include sublingual administration and buccal administration.

In one embodiment of the invention, the subject is subjected to a vaccination protocol comprising daily administration of the vaccine. In another embodiment of the invention the vaccination protocol comprises administration of the vaccine every second day, every third day or every fourth day. For instance, the vaccination protocol comprises administration of the vaccine for a period of more than 4 weeks, preferably more than 8 weeks, more preferably more than 12 weeks, more preferably more than 16 weeks, more preferably more than 20 weeks, more preferably more than 24 weeks, more preferably more than 30 and most preferably more than 36 weeks.

The period of administration may a continuous period. Alternatively, the period of administration is a discontinuous period interrupted by one or more periods of non-administration. Preferably, the (total) period of non-administration is shorter than the (total) period of administration.

In a further embodiment of the invention, the vaccine is administered to the patient once a day. Alternatively, the vaccine is administered to the patient twice a day. The vaccine may be a uni-dose vaccine.

### Liquid vaccine formulation

The liquid vaccine formulation of the invention may further comprise any adjuvant and other excipients suitable for such type of formulation. Such excipients are well-known to the person skilled in the art and include i.a. solvents, emulsifiers, wetting agents, plasticizers, colouring substances, fillers, preservatives, viscosity adjusting agents, buffering agents, pH adjusting agents, isotonicity adjusting agents, mucoadhesive substances, and the like. Examples of formulation strategies are well-known to the person skilled in the art.

The adjuvant may be any conventional adjuvant, including oxygen-containing metal salts, e.g. aluminium hydroxide, heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1β, IL-2, IL-7, IL-12, INFγ), GM-CSF, MDF derivatives, CpG oligonucleotides, LPS, MPL, phosphophazenes, Adju-Phos®, glucan, antigen formulation, liposomes, DDE, DHEA, DMPC, DMPG, DOC/Alum Complex, Freund's incomplete adjuvant, ISCOMs®, LT Oral Adjuvant, muramyl dipeptide, monophosphoryl lipid A, muramyl tripeptide, and phospatidylethanolamine.

Aqueous solutions of oxygen-containing metal salts typically have the form of gels. The concentration of aluminium hydroxide in the formulation is preferably 0.035-1000 mg/ml, more preferably 0.10-100 mg/ml, more preferably 0.25-10 mg/ml, and most preferably 0.5-5 mg/ml. However, the formulation of the invention may also have the form of a highly concentrated gel or gel-like formulation.

In a particular embodiment of the invention the formulation according to the invention further comprises an enhancer. An enhancer is a substance, which increases the bioavailability of a pharmaceutically active substance i) by increasing the penetration of the active substance across the biomembrane by opening the paracellular pathways (tight junctions), i.e. increasing the absorption of the active substance, and/or ii) by the bioadhesive effect of the enhancer, which increases the period of absorption.

Examples of enhancers are an alcohol, e.g. ethanol, chitosan, chitin, propylene glycol, glycerol, dimethyl sulfoxide, dimethyl formamide, dimethyl acetamide, preferably glycerol. The enhancer is preferably present in an amount of from 20 % to 80 % (v/v), more preferably from 30 % (v/v) to 70 % (v/v), and most preferably from 40 % (v/v) to 60 % (v/v).

The formulation of the present invention may be prepared by dissolving the adjuvants and other excipients, if any, in a solvent, preferably water, adding the allergen and allowing the allergen and adjuvants and other excipients to react for a period of time. The reaction period may be from 0.1 to 48 hours, preferably from 12 to 24 hours. The reaction is preferably carried out at a temperature of from 4 to 45 °C, more preferably from 4 to 20 °C.

### Oxygen-containing metal salts

In a preferred embodiment of the invention, the liquid vaccine formulation of the invention comprises an oxygen-containing metal salt adjuvant. Preferably, the metal cation of the oxygen-containing metal salt is selected from the group consisting of Al, K, Ca, Mg, Zn, Ba, Na, Li, B, Be, Fe, Si, Co, Cu, Ni, Ag, Au and Cr.

The anion of the oxygen-containing compound may be any oxygen-containing anion, including an organic or inorganic anion, or a combination of organic and inorganic anions. Examples of suitable oxygen-containing metal salts are e.g. those, wherein the anion is selected from the group consisting of sulphates, hydroxides, phosphates, nitrates, iodates, bromates, carbonates, hydrates, acetates, citrates, oxalates, and tartrates, as well as mixed forms thereof. The oxygen-containing metal salts further comprise coordination complexes. A definition of coordination complexes is given in e.g. The Handbook of Chemistry and Physics 56 Ed., Section B, Chapter 7 (197576).

Within the present context, the expression "mixed forms" is intended to include combinations of the various anions as well as combinations with e. g. chlorides, and sulphides.

Examples of oxygen-containing metal salts according to the invention are aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, zinc sulphate, and barium sulphate.

Most preferred are aluminium hydroxide, aluminium phosphate, aluminium acetate, calcium phosphate, calcium tartrate and zinc sulphate.

The pI of the oxygen-containing metal salt is typically in the range of 2-11. The pI for allergen proteins is typically in the range of 4-9. Preferably, the allergen and oxygen-containing metal salt are selected so that the pI of the allergen is lower than the pl of the oxygen-containing metal salt.

When using e.g. aluminium hydroxide as oxygen-containing metal salt, the concentration of aluminium hydroxide in the formulation is preferably 0.035-1000 mg/ml, more preferably 0.10-100 mg/ml, more preferably 0.25-10 mg/ml, and most preferably 0.5-5 mg/ml. For the other oxygen-containing metal salts, the concentration of the metal salt is preferably 0.035-1000 mg/ml, more preferably 0.35-100 mg/ml, more preferably 0.7-50 mg/ml, and most preferably 1.0-20 mg/ml. The concentration of allergen in the formulation is preferably 0.01-100 mg/ml, more preferably 0.1-10 mg/ml. The ratio of oxygen-containing metal salt to allergen is preferably from 0.1 to 100, more preferably from 1 to 20. The degree of allergen adsorbed to the oxygen-containing metal salt is typically from 5 to 99 %, more preferably from 10 to 99 % of the added amount. The adsorption of allergen to the oxygen-containing metal salt depends on the buffer system and the reaction conditions, including temperature and reaction time, under which the adsorption takes place.

Oxygen-containing metal salts can be characterised by a variety of physical-chemical parameters like adsorption, solubility and dissolution properties, ionic charge measured as the isoelectric point pI (pH where the net charge of the substance is zero for a dissociationable compound), dissociation constants, complex coordination, electronic configurations, valence, bonding orbitals and antibonding orbitals, depot properties, adhesion properties, surface characteristics, particle characteristics, and adjuvanticity.

It is believed that the biologically active substance is adsorbed (or coupled) to the oxygen-containing metal salt, and this adsorption contributes to the efficacy of the vaccine. Several factors may be important or influence the adsorption between the active substance and the oxygen-containing metal salt (see e. g. P. M. Callahan et al., Pharmaceutical Research Vol. 8, No. 7,851-858 (1991), and Vaccine Design. The Subunit and Adjuvant Approach). These factors include pH, the length of time the adsorption reaction is carried out for, mixing conditions, concentrations of the various components in the vaccines, containers, temperature, storage, buffer and excipients. It has further been found that the adsorption of the active substance may be influenced by the net/overall charge of the metal salt and the charge of the active substance, both of which are pH dependent. A further feature believed to be of importance is the solubility of the oxygen-containing metal salts.

Another feature of oxygen-containing salts is the protection of the active substance either by maintaining the ideal pH for the active substance in the microenvironment, thus preventing acid degradation, or by protecting the active substance against enzymatic degradation thereby allowing the substance to be delivered.

Furthermore, some of the oxygen-containing metal salts have a buffer capacity. This may result in an in vivo microenvironment within the vaccine formulation, which protects the active substance from the degradable environment.

A further feature of the oxygen-containing metal salt (s) is their capability to adhere to the mucosal membrane. This is believed to increase absorption of the allergen through the mucosal membrane.

For several of the oxygen-containing metal salts (e. g. Al(OH)₃, AlPO₄, Ca₃PO₄) the particle size range is between 0.5 and 15 µm.

### DEFINITIONS

The term "oromucosal administration" refers to a route of administration where the dosage form is placed under the tongue or anywhere else in the oral cavity to allow the active ingredient to come in contact with the mucosa of the oral cavity or the pharynx of the patient in order to obtain a local or systemic effect of the active ingredient. An example of an oromucosal administration route is sublingual administration.

The term "sublingual administration" refers to a route of administration, where a dosage form is placed underneath the tongue in order to obtain a local or systemic effect of the active ingredient.

The term "no up-dosing" means that full doses are used from the first administration and onwards, wherein the term "full dose" means the dose used in the continued treatment.

The term "liquid vaccine formulation" means any liquid or fluid formulation, including solutions, suspensions and gels with low and high viscosity.

The term "SQ-u" means Standardised Quality-Unit: The SQ-u is determined in accordance with ALK-Abelló A/S's "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. The precise allergen amount can be determined by means of immunoassay i.e. total major allergen content and total allergen activity.

The term "treating" means partly of wholly curing, alleviating symptoms or inhibiting causes of symptoms.

The term "preventing" means any type of prophylactic treatment.

In the field of allergy extracts, there is no international accepted standardisation method. A number of different units of extract strength i.e. bio-potency exist. The methods employed and the units used normally measure the allergen content and biological activity. Examples hereof are SQ-Units (Standardised Quality units), BAU (Biological Allergen Units), BU (biological units), UM (Units of Mass), IU (International Units) and IR (Index of Reactivity). Hence, if extracts of origins other than those disclosed herein are used, they need to be standardised against extract disclosed herein in order to determine their potency in SQ units or any of the above mentioned units. The subject matter is dealt with in "Allergenic extracts", H. Ipsen et al, chapter 20 in Allergy, principle and practise (Ed. S. Manning) 1993, Mosby-Year Book, St. Louis and Løwenstein H. (1980) Arb Paul Ehrlich Inst 75:122.

The bio-potency, i.e. the in vivo allergenic activity, of a given extract depends on a number of factors, the most important being the content of major allergens in the extract, which varies with the composition of the biological source material.

The amount of allergen extract in grams to be used for obtaining a desired bio-potency varies with the type of extract in question, and for a given type of extract the amount of allergen extract varies from one batch to another with the actual bio-potency of the extract.

For a given batch of extract, the amount of allergen extract in grams to be used for obtaining a desired bio-potency may be determined using the following procedure:
a) The bio-potency of various amounts of a reference extract is determined using one or more immunological in vivo tests to establish a relationship between bio-potency and amount of reference extract. Examples of the said immunological in vivo tests are Skin Prick Test (SPT), Conjunctival Provocation Test (CPT), Bronchial Challenge with Allergen (BCA) and various clinical trials in which one or more allergy symptoms is monitored, see for example e.g. Haugaard et al., J Allergy Clin Immunol, Vol. 91, No. 3, pp 709-722, March 1993.
b) On the basis of the established relationship between bio-potency and reference extract, the bio-potency of one or more relevant doses for use in the dosage forms of the invention is selected with due consideration to a balance of the factors of i) the effect of treating or alleviating symptoms of allergy, ii) side effects recorded in the immunological in vivo tests, and iii) the variability of i) and ii) from one individual to another. The balancing is done to obtain a maximal adequate therapeutic effect without experiencing an unacceptable level of side effect. The way of balancing the factors are well known to those skilled in the art
   The bio-potency of the one or more relevant doses found may be expressed in any biopotency unit available, such as SQ units, BAU, IR units, IU, cf. above.
c) From the reference extract one or more bio-potency reference standard extracts is prepared and, if used, the bio-potency unit values of the reference standard extracts are calculated on the basis of the bio-potency unit value allocated to the one or more relevant doses, e.g. such a standard for BAU can be obtained from FDA as illustrated below.
d) For the reference standard extracts of each extract type, a number of parameters for evaluating the bio-potency of extracts are selected. Examples of such evaluation parameters are total allergenic activity, the amount of defined major allergens and overall molecular composition of the extract. The total allergenic activity may be measured using an in vitro competitive immunoassay, such as ELISA and MagicLite® luminescence immunoassay (LIA), using a standardised antibody mixture raised against the extract obtained using standard methods, e.g. antibodies raised in mouse or rabbit, or a pool of allergic patients sera. The content of major allergens may e.g. be quantified by rocket immuno-electrophoresis (RIE) and compared to the reference standards. The overall molecular composition may be examined using e.g. crossed immunoelectrophoresis (CIE) and sodium dodecyl sulphate polyacrylamide gel electrophoresis (SDS-PAGE).
e) For a given batch of extract of unknown bio-potency (test extract), the amount of extract to be used for obtaining a desired bio-potency level (effective dose for use in the solid dosage form according to the present invention) may be determined as follows: For each evaluation parameter selected, the test extract is compared with the reference standard extracts using the relevant measurement methods as described above, and on the basis of the measurement results the amount of extract having the desired bio-potency is calculated.

SQ-Unit: The SQ-Unit is determined in accordance with ALK-Abelló A/S's "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. The precise allergen amount can be determined by means of immunoassay i.e. total major allergen content and total allergen activity.

STU stands for Specific Treatment Unit, and STU is an empirical unit based on the biologicall activity of the respective allergen extract used for allergen specific allergy vaccination. 1000 STU corresponds to the potency of the highest maintenance dose used in a liquid formulation for SLIT, wherein the maintenance dose is the dose recommended in specific allergy vaccination to obtain the best safety/efficacy ratio in the average population of allergic patients.

BAU (Biological Allergen Units) is biological potency units as determined according to the requirements of the FDA for allergen product described in "Quantitative determination of relative potency of allergenic extracts"

("Methods of the allergen products testing Laboratory" "ELISA competition assay". Page 15, #49N-0012, FDA, October 1993). A dose of 100,000 SQ-Units containing grass extract equals a content of 2600-4700 BAU according to the method above. Likewise, other extracts can be assessed according to the method above.

### EXAMPLES

### Example 1: Sublingual immunotherapy (SLIT) (SLITone^{®}) without updosing - a new treatment schedule

### Introduction

During the last years several studies have been carried out with sublingual immunotherapy (SLIT) yielding evidence of efficacy combined with a favourable safety profile (1).

In order for patients to obtain a high level of effect of a self-administered treatment like SLITone^{®}, compliance must be high (2). Given that one of the cornerstones for compliance is convenience, it is relevant to improve this feature of the product. The excellent tolerance of SLIT has led to considering the possibility of eliminating the updosing phase. This would improve patient convenience, and may thereby increase compliance and potentially increase the treatment effect. This study was designed to evaluate the tolerability of SLITone^{®} without updosing in patients with allergy to mites or grasses.

### Material and methods

### Medicament

SLITone® (ALK-Abelló) is a monodose liquid formulation containing 0.2 ml solution containing an allergen dose of 200 STU, wherein the solution is 50 % (v/v) glycerol and 50 % (v/v) water.

### Patients

A total of 135 patients were included (66 grass- and 69 mite allergic patients), aged between 7 and 55 years, all diagnosed with moderate-severe rhinitis or rhinoconjunctivitis with/without asthma symptoms, due to sensitivity to either mites or grasses. The diagnosis was made by a positive skin-prick test (prick-test, ALK-Abelló, S.A., 100 BU/ml) and/or positive IgE (CAP ≥ pharmacy class 2). In accordance with WHO recommendations (3), the trial excluded patients where treatment with immunotherapy was contraindicated, those sensitive to allergens other than those evaluated in the trial, and those who had received immunotherapy previously.

### Study design

The study was a randomised, multi-centre, double blind, placebo-controlled, parallel-group study. The total duration was 90 days.

Patients were allocated in two groups. Group 1 (N=69) received active treatment throughout the study. This included 10 days updosing (T1), 20 days maintenance treatment (T2), and two consecutive periods of 30 days duration (T3 and T4) on maintenance treatment. Group 2 (N=66) received placebo updosing during T1, placebo maintenance during T2, and initiated active treatment at the beginning of T3 and continued active treatment until the end of the study (T4). Data was collected at baseline and at T1-T4. Each patient received a card where any clinical incidents related to each treatment dose were noted. The investigator noted the adverse events on the Case Report Forms.

The study was double blinded and the blinding was not broken at any time until after the study was finalised. All patients signed an informed consent form before commencing their participation in the trial. The study was approved by all Ethics Committees of the participating centres, as well as the Health Authorities.

### Treatment

SLITone^{®} (ALK-Abelló) was administered sublingually and composed of either mite extract (*Dermatophagoides pteronyssinus or Dermatophagoides pteronyssinus* + *Dermatophagoides farinae*) or an extract of a mixture of grasses *(Dactylis glomerata, Festuca pratensis, Lolium perenne, Phleum pratense, Poa pratensis, Secale cereale*)*.* Treatment consisted of 10 days updosing with a multi-dose vial (beginning with 1 drop and increasing to 10 drops) and a maintenance phase with monodose containers. Administration was once daily. The dose of the major allergens administered in each monodose container was 2 µg of major allergen 5 (for grasses) and 0.8/0.4 µg of Der 1 / Der 2 (for mites).

The appearance, smell and taste of the placebo treatment was similar to the active treatment.

### Statistics

Descriptive statistics was used. The description included number of patients reporting adverse events (AE's) (N), the percentage of patients experiencing AEs (%), and the number of AE's (E).

### Results

Of the 135 patients included, 123 completed the study. Patient characteristics are shown in Table 1. The age of patients ranged from 7 to 55 years with a mean age of 22 in both treatment groups.

In group 1, 36 patients received grass extract and 33 received mite extract compared to 33 receiving grass extract and 33 receiving mite extract in group 2. A total of 229 AE's were reported; 157 AE's (68 and 89, in group 1 and 2, respectively) were evaluated as related to the treatment by the investigator and included in the analysis. The most frequently reported AE's (> 5% of patients in any group) related to treatment were oral itching, ocular itching, rhinitis, and sublingual oedema (Table 2); in total these accounted for 57.3% of all related AE's. Oral itching was the most frequently reported AE related to treatment; 16 patients (23.2%) reported 28 events of oral itching in group 1 compared to 21 patients (31.8%) reporting 39 events of oral itching in group 2. Oral itching was the only AE that was reported by > 10% of patients in any group and as a total accounted for 42.7% of all events.

The total number of SLIT doses administered was 6258 and 6081 in group 1 and 2, respectively. The rate of oral itching per dose administered by time (T1 - T4) is shown in Table 3.

The most frequently reported AE's related to treatment for patients ≤ 14 years (children) and for patients > 14 years (adolescents and adults) are shown in Table 4.

A total of 5 patients withdrew due to adverse events. In group 1 three subjects withdrew due to headache at T1; asthma, rhinitis and pruritus at T1; and sublingual oedema at T4, respectively. In group 2 two subjects withdrew due to dyspnoea and cough at T2, and tongue and mouth oedema at T4, respectively.

### Discussion

The purpose of this randomised double blind placebo controlled parallel group study was to investigate the tolerability of SLIT administered daily without an initial updosing phase. The results presented here indicate that SLITone^{®} (grasses and mites) is well tolerated, and that tolerance is not influenced by omission of the updosing phase. Overall the number of patients reporting AE's was slightly higher in group 2 (receiving placebo updosing) compared to group 1 (receiving active updosing), and so was the number of events. However, it should be noted that in group 2, a substantial number of events were reported during the placebo treatment (T1 and T2). The most frequently reported AE was oral itching. Oral itching is an expected and well known side effect of sublingual immunotherapy, and has been reported to the same extent or more frequently by other authors (4-7).

The use of updosing in SLIT is empirical and based on tradition and is probably adapted from the necessary stepwise dose increase used in subcutaneous immunotherapy. Data from previous clinical trials (4;6) and pharmacovigilance studies (7) indicate excellent tolerance of SLIT, but it was not until recently that the impact of the updosing phase on tolerability was put up for discussion. The possible impact of updosing in SLIT is speculative. Assuming that the effect of updosing in SLIT is for the patient to be able to administer the maintenance dose safely after updosing, the data presented here prove this hypothesis to be wrong. The number of patients in group 2 reporting oral itching at T3 (when active treatment was initiated as maintenance dose) was higher than in group 1 at T1, but is was similar to the number of patients in group 1 reporting oral itching at T2, at which time the hypothesised effect of the updosing phase on the patient's tolerability warranted a lower number. The rate of oral itching per administered dose also indicates that these events were reported equally frequent in group 1 at T2 and group 2 at T3. Furthermore, when comparing the first 30 days of active treatment in both groups (T1+T2 for group 1 and T3 for group 2), the accumulated rate of oral itching per administered dose was 0.83% and 0.74% for group 1 and group 2, respectively. The short duration of this study does not allow for any firm conclusions on whether or not reporting of oral itching will decline over time. However, the relatively few withdrawals observed indicate that the patients did not consider the intensity and duration of oral itching bothersome.

Sublingual oedema was reported more frequently in group 2 than in group 1.

This type of event was in group 2 reported when active treatment was administered (T3 and T4), and resolved within 1 hour to 3 days. One patient withdrew from the study due to this AE at T4 after a minimum of 30 days active treatment. Sublingual oedema was also reported in group 1 at T4. When comparing oral itching in children and in adolescents and adults it appears from the data that oral itching is reported more frequently in children than in adolescents and adults. The reasons for this are not apparent, but it remains a topic for further investigation. However, the number of withdrawals among children was not different from that of adolescents and adults. Furthermore, the withdrawals among children (N=2) were not related to oral itching.

In conclusion these study results indicate that SLITone^{®} (mites and grasses) is well tolerated, and that treatment can be initiated safely without an updosing phase.

### References

(1) Wilson DR, Torres LM, Durham SR. Sublingual immunotherapy for allergic rhinitis: systematic review and meta-analysis. Allergy 2005; 60(1):4-12.
(2) Osterberg L, Blaschke T. Adherence to medication. N Engl J Med 2005; 353(5):487-497.
(3) Bousquet J, Lockey RF, Malling HJ. WHO Position Paper. Allergen immunotherapy: Therapeutic vaccines for allergic diseases. Allergy 1998; 53(44):1-42.
(4) Pajno GB, Morabito L, Barberio G, Parmiani S. Clinical and immunologic effects of long-term sublingual immunotherapy in asthmatic children sensitized to mites: a double-blind, placebo-controlled study. Allergy 2000; 55(9):842-849.
(5) Canonica GW, Passalacqua G. Noninjection routes for immunotherapy. J Allergy Clin Immunol 2003; 111(3):437-448.
(6) Quirino T, lemoli E, Siciliani E, Parmiani S, Milazzo F. Sublingual versus injective immunotherapy in grass pollen allergic patients: a double blind (double dummy) study. Clin Exp Allergy 1996; 26(11):1253-1261.
(7) Di Rienzo V, Pagani A, Parmiani S, Passalacqua G, Canonica GW. Post-marketing surveillance study on the safety of sublingual immunotherapy in pediatric patients. Allergy 1999; 54(10):1110-1113.

**Table 1: Patient characteristics**

| Total n = 135 | | Group 1 | Group 2 |
|---|---|---|---|
| | | n = 69 | n = 66 |
| Sex | Male | 38 (55%) | 31 (47%) |
| | Female | 31 (45%) | 35 (53%) |
| Age (years) | Mean (SD) | 22.6 (±11.2) | 22.1 (±9.7) |
| | ≤ 14 years | 21 (30%) | 19 (29%) |
| | >14 years | 48 (70%) | 47 (71%) |
| Diagnosis | Rhinitis | 33 (48%) | 32 (48%) |
| | Rhinits + Asthma | 36 (52%) | 34 (52%) |
| Withdrawals | Total | 6 | 6 |
| | Related to AEs | 3 | 2 |

**Table 2: Most frequently reported AE's (> 5% in any group) related to treatment**

| n = 135 | | Group 1 n=69 | Group 2 n=66 |
|---|---|---|---|
| | | N (%) E | N (%) E |
| Oral itching | T1 | 4 (5.9%) 5 | 8 (12.1%) 8 |
| | T2 | 12 (18.2%) 13 | 5 (7.6%) 6 |
| | T3 | 5 (7.7%) 5 | 13 (20.6%) 15 |
| | T4 | 5 (7.8%) 5 | 9 (14.8%) 10 |
| | Total | 16 (23.2%) 28 | 21 (31.8%) 39 |
| Ocular itching | T1 | 4 (5.9%) 4 | 1 (1.5%) 1 |
| | T2 | 1 (1.5%) 1 | 0 (0.0%) 0 |
| | T3 | 0 (0.0%) 0 | 1 (1.6%) 1 |
| | T4 | 0 (0.0%) 0 | 0 (0.0%) 0 |
| | Total | 5 (7.2%) 5 | 1 (1.5%) 2 |
| Rhinitis | T1 | 1 (1.5%) 1 | 1 (1.5%) 1 |
| | T2 | 0 (0.0%) 0 | 1 (1.5%) 3 |
| | T3 | 1 (1.5%) 1 | 0 (0.0%) 0 |
| | T4 | 0 (0.0%) 0 | 1 (1.6%) 1 |
| | Total | 2 (2.9%) 2 | 4 (6.1 %) 5 |
| Sublingual oedema | T1 | 0 (0.0%) 0 | 0 (0.0%) 0 |
| | T2 | 0 (0.0%) 0 | 0 (0.0%) 0 |
| | T3 | 1 (1.5%) 1 | 3 (4.8%) 3 |
| | T4 | 1 (1.7%) 1 | 4 (6.6%) 4 |
| | Total | 1 (1.4%) 2 | 4 (6.1%) 7 |

The number of patients (N) reporting the most frequently reported adverse events (by > 5 % of patients in any group), the percentage of the group population (%), the number of events (E), and the time of reporting in both treatment groups. Group 1 received active treatment from T1-T4, group 2 received placebo during T1 and T2. Total number of patients is corrected for patients repeatedly reporting the same type of adverse events.

**Table 3. Rate of oral itching per dose administered (%) by time of reporting.**

| | Group 1 | | | Group 2 | | |
|---|---|---|---|---|---|---|
| Time | E | Doses | Rate (%) | E | Doses | Rate (%) |
| T1 | 5 | 875 | 0.57 | 8 | 811 | 0.99 |
| T2 | 13 | 1288 | 1.01 | 6 | 1238 | 0.48 |
| T3 | 5 | 2039 | 0.25 | 15 | 2027 | 0.74 |
| T4 | 5 | 2056 | 0.24 | 10 | 2005 | 0.50 |
| Total | 28 | 6258 | 0.45 | 39 | 6081 | 0.64 |

| | | | | | | |
|---|---|---|---|---|---|---|
| E: Number of events of oral itching | | | | | | |

Rate of event per dose administered (%) in the most frequently reported adverse event (oral itching) by time of reporting.

**Table 4. Number of patients with most frequently reported adverse events related to treatment by age**

| | Group 1 | Group 2 |
|---|---|---|
| ≤14 years | N (%) | N (%) |
| | n=21 | n=19 |
| Oral itching | 8 (38.1 %) | 8 (42.1 %) |
| Ocular itching | 1 (4.8%) | 1 (5.3%) |
| Rhinitis | 2 (9.5%) | 3 (15.8%) |
| Sublingual oedema | 0 (0.0%) | 2 (10.5%) |
| > 14 years | n = 48 | n = 47 |
| Oral itching | 8 (16.7%) | 13 (27.7%) |
| Ocular itching | 4 (8.3%) | 0 (0.0%) |
| Rhinitis | 0 (0.0%) | 1 (2.1%) |
| Sublingual oedema | 1 (2.1%) | 2 (4.3%) |

Number of patients with the most frequently reported adverse event related to treatment by age.

## Claims

1. An allergen for use in prevention or treatment of allergy in a human subject by oromucosal administration of a liquid vaccine formulation in a dosage regimen comprising no up-dosing.

2. The allergen for use according to claim 1, wherein the concentration of major allergen in a mono-dose intended for daily administration is from 0.05 to 50 µg, more preferably from 0.05 µg to 30 µg, more preferably from 0.06 µg to 25 µg, more preferably from 0.07 µg to 20 µg, more preferably from 0.08 µg to 15 µg, more preferably from 0.09 µg to 10 µg and most preferably from 0.1 µg to 7 µg.

3. The allergen for use according to claim 1 or 2, which is selected from the group consisting of a tree pollen allergen, a grass pollen allergen, a house dust mite allergen, a storage mite allergen, a weed allergen, a mould allergen, a cat allergen and a dog allergen.

4. The allergen for use according to any of the preceding claims, wherein the oromucosal administration is sublingual administration.

5. The allergen for use according to any of the preceding claims, which is formulated with an enhancer.

6. The allergen for use according to claim 5, wherein the enhancer is glycerol.

7. An allergen for use according to any of the preceding claims, wherein the vaccine further comprises an adjuvant selected from the group of oxygen-containing metal salts.

8. An allergen for use according to claim 7, wherein the oxygen-containing metal salt is selected form the group consisting of aluminium hydroxide, aluminium phosphate and calcium phosphate.

9. An allergen for use according to claim 8, wherein the oxygen-containing metal salt is aluminium hydroxide.

10. An allergen for use according to claim 9, wherein the concentration of oxygen-containing metal salt is 0.035-1000 mg/ml, more preferably 0.10-100 mg/ml, more preferably 0.25-10 mg/ml, and most preferably 0.5-5 mg/ml.

11. The allergen for use according to claim 1, which is a tree pollen allergen, which is formulated together with glycerol, and the oromucosal administration is sublingual administration.

12. The allergen for use according to claim 11, wherein the tree pollen allergen is a birch pollen allergen.

13. The allergen for use according to claim 1, which is a grass pollen allergen, which is formulated together with glycerol, and the oromucosal administration is sublingual administration.

14. The allergen for use according to claim 1, which is a house dust mite allergen, which is formulated together with glycerol, and the oromucosal administration is sublingual administration.

15. The allergen for use according to claim 1, wherein the allergen is a storage mite allergen, which is formulated together with glycerol, and the oromucosal administration is sublingual administration.

16. The allergen for use according to claim 1, which is a weed allergen, which is formulated together with glycerol, and the oromucosal administration is sublingual administration.

17. The allergen for use according to claim 1, which is a mould allergen, which is formulated together with glycerol, and the oromucosal administration is sublingual administration.

18. The allergen for use according to claim 1, which is a cat allergen, which is formulated together with glycerol, and the oromucosal administration is sublingual administration.

19. The allergen for use according to claim 1, which is a dog allergen, which is formulated together with glycerol, and the oromucosal administration is sublingual administration.

## Patentansprüche

1. Allergen zur Verwendung bei der Prävention oder Behandlung einer Allergie bei einem humanen Subjekt durch oromukosale Verabreichung einer flüssigen Vakzinformulierung in einem Dosierungsregime, welches keine Dosierungsanpassung nach oben umfasst.

2. Allergen zur Verwendung nach Anspruch 1, wobei die Konzentration des Hauptallergens in einer Monodosis, die zur täglichen Verabreichung bestimmt ist, von 0,05 bis 50 µg, bevorzugter von 0,05 µg bis 30 µg, bevorzugter von 0,06 µg bis 25 µg, bevorzugter von 0,07 µg bis 20 µg, bevorzugter von 0,08 µg bis 15 µg, bevorzugter von 0,09 µg bis 10 µg und am bevorzugtesten von 0,1 µg bis 7 µg beträgt.

3. Allergen zur Verwendung nach Anspruch 1 oder 2, welches ausgewählt ist aus der Gruppe bestehend aus einem Baumpollenallergen, einem Graspollenallergen, einem Hausstaubmilbenallergen, einem Vorratsmilbenallergen, einem Unkrautallergen, einem Schimmelallergen, einem Katzenallergen und einem Hundeallergen.

4. Allergen zur Verwendung nach einem der vorhergehenden Ansprüche, wobei die oromukosale Verabreichung eine sublinguale Verabreichung ist.

5. Allergen zur Verwendung nach einem der vorhergehenden Ansprüche, welches mit einem Wirkverstärker formuliert ist.

6. Allergen zur Verwendung nach Anspruch 5, wobei der Wirkverstärker Glycerol ist.

7. Allergen zur Verwendung nach einem der vorhergehenden Ansprüche, wobei das Vakzin ferner ein Adjuvans ausgewählt aus der Gruppe von sauerstoffhaltigen Metallsalzen umfasst.

8. Allergen zur Verwendung nach Anspruch 7, wobei das sauerstoffhaltige Metallsalz ausgewählt ist aus der Gruppe bestehend aus Aluminiumhydroxid, Aluminiumphosphat und Calciumphosphat.

9. Allergen zur Verwendung nach Anspruch 8, wobei das sauerstoffhaltige Metallsalz Aluminiumhydroxid ist.

10. Allergen zur Verwendung nach Anspruch 9, wobei die Konzentration des sauerstoffhaltigen Metallsalzes 0,035-1000 mg/ml, bevorzugter 0,0-100 mg/ml, bevorzugter 0,25-10 mg/ml und am bevorzugtesten 0,5-5 mg/ml beträgt.

11. Allergen zur Verwendung nach Anspruch 1, bei welchem es sich um ein Baumpollenallergen handelt, das zusammen mit Glycerol formuliert ist, und wobei die oromukosale Verabreichung eine sublinguale Verabreichung ist.

12. Allergen zur Verwendung nach Anspruch 11, wobei das Baumpollenallergen ein Birkenpollenallergen ist.

13. Allergen zur Verwendung nach Anspruch 1, bei welchem es sich um ein Graspollenallergen handelt, das zusammen mit Glycerol formuliert ist, und wobei die oromukosale Verabreichung eine sublinguale Verabreichung ist.

14. Allergen zur Verwendung nach Anspruch 1, bei welchem es sich um ein Hausstaubmilbenallergen handelt, das zusammen mit Glycerol formuliert ist, und wobei die oromukosale Verabreichung eine sublinguale Verabreichung ist.

15. Allergen zur Verwendung nach Anspruch 1, wobei das Allergen ein Vorratsmilbenallergen ist, das zusammen mit Glycerol formuliert ist, und wobei die oromukosale Verabreichung eine sublinguale Verabreichung ist.

16. Allergen zur Verwendung nach Anspruch 1, bei welchem es sich um ein Unkrautallergen handelt, das zusammen mit Glycerol formuliert ist, und wobei die oromukosale Verabreichung eine sublinguale Verabreichung ist.

17. Allergen zur Verwendung nach Anspruch 1, bei welchem es sich um ein Schimmelallergen handelt, das zusammen mit Glycerol formuliert ist, und wobei die oromukosale Verabreichung eine sublinguale Verabreichung ist.

18. Allergen zur Verwendung nach Anspruch 1, bei welchem es sich um ein Katzenallergen handelt, das zusammen mit Glycerol formuliert ist, und wobei die oromukosale Verabreichung eine sublinguale Verabreichung ist.

19. Allergen zur Verwendung nach Anspruch 1, bei welchem es sich um ein Hundeallergen handelt, das zusammen mit Glycerol formuliert ist, und wobei die oromukosale Verabreichung eine sublinguale Verabreichung ist.

## Revendications

1. Allergène destiné à être utilisé dans la prévention ou le traitement d'une allergie chez un sujet humain par administration oromuqueuse d'une formulation de vaccin liquide en un régime posologique ne comprenant pas d'augmentation des doses.

2. Allergène destiné à être utilisé selon la revendication 1, dans lequel la concentration en allergène majeur dans une monodose destinée à une administration quotidienne est de 0,05 à 50 µg, de préférence de 0,05 µg à 30 µg, de préférence encore de 0,06 µg à 25 µg, de manière davantage préférée de 0,07 µg à 20 µg, de manière encore davantage préférée de 0,08 µg à 15 µg, de manière toujours davantage préférée de 0,09 µg à 10 µg et idéalement de 0,1 µg à 7 µg.

3. Allergène destiné à être utilisé selon la revendication 1 ou 2, qui est choisi dans le groupe constitué d'un allergène de pollen d'arbre, d'un allergène de pollen de graminée, d'un allergène de pollen d'acarien dermatophagoïde, d'un allergène d'acarien de stockage, d'un allergène de mauvaise herbe, d'un allergène de moisissure, d'un allergène de chat et d'un allergène de chien.

4. Allergène destiné à être utilisé selon l'une quelconque des revendications précédentes, dans lequel l'administration oromuqueuse est une administration sublinguale.

5. Allergène destiné à être utilisé selon l'une quelconque des revendications précédentes, qui est formulé avec un amplificateur.

6. Allergène destiné à être utilisé selon la revendication 5, dans lequel l'amplificateur est le glycérol.

7. Allergène destiné à être utilisé selon l'une quelconque des revendications précédentes, dans lequel le vaccin comprend en outre un adjuvant choisi dans le groupe constitué des sels de métaux contenant de l'oxygène.

8. Allergène destiné à être utilisé selon la revendication 7, dans lequel le sel de métal contenant de l'oxygène est choisi dans le groupe constitué de l'hydroxyde d'aluminium, du phosphate d'aluminium et du phosphate de calcium.

9. Allergène destiné à être utilisé selon la revendication 8, dans lequel le sel de métal contenant de l'oxygène est l'hydroxyde d'aluminium.

10. Allergène destiné à être utilisé selon la revendication 9, dans lequel la concentration en sel de métal contenant de l'oxygène est de 0,035 à 1 000 mg/mL, de préférence de 0,10 à 100 mg/mL, de manière davantage préférée de 0,25 à 10 mg/mL, et idéalement de 0,5 à 5 mg/mL.

11. Allergène destiné à être utilisé selon la revendication 1, qui est un allergène de pollen d"arbre, qui est formulé avec du glycérol, et l'administration oromuqueuse est une administration sublinguale.

12. Allergène destiné à être utilisé selon la revendication 11, dans lequel l'allergène de pollen d'arbre est un allergène de pollen de bouleau.

13. Allergène destiné à être utilisé selon la revendication 1, qui est un allergène de pollen de graminée, qui est formulé avec du glycérol, et l'administration oromuqueuse est une administration sublinguale.

14. Allergène destiné à être utilisé selon la revendication 1, qui est un allergène d'acarien dermatophagoïde, qui est formulé avec du glycérol, et l'administration oromuqueuse est une administration sublinguale.

15. Allergène destiné à être utilisé selon la revendication 1, qui est un allergène d'acarien de stockage, qui est formulé avec du glycérol, et l'administration oromuqueuse est une administration sublinguale.

16. Allergène destiné à être utilisé selon la revendication 1, qui est un allergène de mauvaise herbe, qui est formulé avec du glycérol, et l'administration oromuqueuse est une administration sublinguale.

17. Allergène destiné à être utilisé selon la revendication 1, qui est un allergène de moisissure, qui est formulé avec du glycérol, et l'administration oromuqueuse est une administration sublinguale.

18. Allergène destiné à être utilisé selon la revendication 1, qui est un allergène de chat, qui est formulé avec du glycérol, et l'administration oromuqueuse est une administration sublinguale.

19. Allergène destiné à être utilisé selon la revendication 1, qui est un allergène de chien, qui est formulé avec du glycérol, et l'administration oromuqueuse est une administration sublinguale.
